# EUROPEAN PATENT APPLICATION

(11) **EP 4 129 179 A1**
(43) Date of publication of application: **08.02.2023**
(21) Application number: 21781632.1
(22) Date of filing: 29.03.2021
(51) Int. Cl.: A61B 5/332

(54) **BIOSENSOR**

(30) Priority: 30.03.2020 JP 2020059657
(71) Applicant: Nitto Denko Corporation, Ibaraki-shi, Osaka 567-8680 (JP)
(72) Inventor: KONDO, Sota, Ibaraki-shi, Osaka 567-8680 (JP)
(74) Representative: Hoffmann Eitle
(86) International application number: PCT/JP2021/013252
(87) International publication number: WO 2021/200807

(57) **Abstract**

A biosensor, which operates by power supplied from a battery, includes an electrode portion positioned on at least a side of one terminal of the battery; and a conductive adhesive tape having conductivity provided between the one terminal and the electrode portion. A fluctuation width of a resistance value of the conductive adhesive tape is 1.60 Ω or less in absolute value when an iron ball having a weight of 33 g is dropped vertically from a height of 30 cm to apply a load to a surface of the conductive adhesive tape.

## Description

### [Technical Field]

The present invention relates to a biosensor.

### [Background Art]

In a medical institution such as a hospital or a clinic, a nursing facility, or a home, a biosensor for measuring biological information such as an electrocardiogram, pulse wave, electroencephalogram, or myoelectricity is used. The biosensor is provided with a bio-electrode for acquiring biological information of a subject by contacting with a living body, and when measuring the biological information, the biosensor is affixed to a skin of the subject, and the bio-electrode is brought into contact with the skin of the subject. The biological information is measured by acquiring an electric signal relating to the biological information with a bio-electrode.

As such a biosensor, for example, a patch-type biological device which includes a wiring board stored in a storage space defined by an upper exterior body and a lower exterior body, and a coin battery mounted on the wiring board, power being supplied from the coin battery to a wireless communication unit, and data acquired by the wireless communication unit being transmitted, is disclosed (See, for example, Patent Document 1.).

### [Prior art document]

### [Patent Document]

[Patent Document 1] Japanese Unexamined Patent Application Publication No. 2019-203881

### [Summary of the Invention]

### [Problems to be Solved by the Invention]

In a biosensor having a battery such as a coin battery or a controller for controlling an operation of the biosensor inside, as in the case of the conventional patch-type biosensor, when the biosensor is used, there is a possibility that the resistance of a conductive member such as a wiring arranged between the battery and the controller rises due to contact with pressure to a skin of the subject, movement of the living body (body movement), or the like, and the voltage supplied from the battery drops. In particular, when the supply voltage from the battery falls below an operable voltage of the controller, there is a case where the operation of the biosensor is stopped and the biological information cannot be measured.

An object of the present invention is to provide a biosensor in which a voltage can be stably supplied from a battery to a controller.

### [Means for Solving the Problem]

According to an aspect of the present invention, a biosensor, which operates by power supplied from a battery, includes an electrode portion positioned on at least a side of one terminal of the battery; and a conductive adhesive tape having conductivity provided between the one terminal and the electrode portion, a fluctuation width of a resistance value of the conductive adhesive tape being 1.60 Ω or less in absolute value when an iron ball having a weight of 33 g is dropped vertically from a height of 30 cm to apply a load to a surface of the conductive adhesive tape.

### [Effect of the Invention]

According to an aspect of the present invention, in the biosensor a voltage can be stably supplied from the battery to the controller.

### [Brief Description of Drawings]

[Figure 1] FIG. 1 is a perspective view showing a configuration of a biosensor according to an embodiment of the present invention.
[Figure 2] FIG. 2 is an exploded perspective view of FIG. 1.
[Figure 3] FIG. 3 is a cross-sectional view cut along I-I in FIG. 1.
[Figure 4] FIG. 4 is a plan view showing a configuration of a sensor portion.
[Figure 5] FIG. 5 is an exploded perspective view of a part of the sensor portion of FIG. 4.
[Figure 6] FIG. 6 is an explanatory diagram showing a layout of the sensor portion of FIG. 4.
[Figure 7] FIG. 7 is a partial cross-sectional view cut along II-II in FIG. 4.
[Figure 8] FIG. 8 is a diagram showing an example of another configuration of a first conductive adhesive tape and a second conductive adhesive tape.
[Figure 9] FIG. 9 is a diagram showing an example of yet another configuration of the first conductive adhesive tape and the second conductive adhesive tape.
[Figure 10] FIG. 10 is an explanatory diagram showing a state in which the biosensor is affixed to a skin of a living body (subject).
[Figure 11] FIG. 11 is an explanation diagram illustrating a three-point bending test.
[Figure 12] FIG. 12 is a diagram showing a relationship between time and voltage in the biosensor of Example 1.
[Figure 13] FIG. 13 is a diagram showing a relationship between time and voltage in the biosensor of Example 2.
[Figure 14] FIG. 14 is a diagram showing a relationship between time and voltage in the biosensor of Example 3.
[Figure 15] FIG. 15 is a diagram showing a relationship between time and voltage in the biosensor of Example 4.
[Figure 16] FIG. 16 is a diagram showing a relationship between time and voltage in the biosensor of Example 5.
[Figure 17] FIG. 17 is a diagram showing a relationship between time and voltage in the biosensor of Example 6.
[Figure 18] FIG. 18 is a diagram showing a relationship between time and voltage in the biosensor of Comparative example 1.
[Figure 19] FIG. 19 is a diagram showing a relationship between time and voltage in the biosensor of Comparative example 2.
[Figure 20] FIG. 20 is a diagram showing a relationship between time and voltage in the biosensor of Comparative example 3.

### [Mode for carrying out the Invention]

In the following, embodiments of the present disclosure will be described in detail. To facilitate understanding of the description, in each drawing, to the same elements, the same reference numeral will be assigned, and a duplicate explanation may be omitted. Moreover, a scale of each member in the drawings may be different from the actual scale. In the specification of the present application, a three-dimensional orthogonal coordinate system in three axes (in an X-axis direction, a Y-axis direction, and a Z-axis direction) is used. A transverse direction of the biosensor is set to be the X-axis direction, the longitudinal direction of the biosensor is set to be the Y-axis direction, and the height direction (in the thickness direction) of the biosensor is set to be the Z-axis direction. A direction opposite to the side (sticking side) on which the biosensor is affixed to the living body (subject) is set to be a +Z-axis direction, and the side (sticking side) on which the biosensor is affixed to the living body (subject) is set to be a -Z-axis direction. In the following description, for convenience of illustration, the +Z-axis side will be referred to as an upper side or above, and the -Z-axis side will be referred to as a lower side or below. However, they do not represent a universal vertical relationship. In this specification, a hyphen "-" indicating a numerical value range means to include numerical values described before and after the hyphen as the lower limit value and the upper limit value, unless otherwise stated.

### <Biosensor>

A biosensor according to the present embodiment will be described. In the present embodiment, as an example, a case of a patch-type biosensor brought into contact with a living body to measure biological information will be described. The term "living body" includes a human body (human) and an animal, such as a cow, a horse, a pig, a chicken, a dog, and a cat. The biosensor is affixed to a part of the living body (for example, skin, scalp, or forehead). The biosensor can be suitably used for a living body, especially a human body.

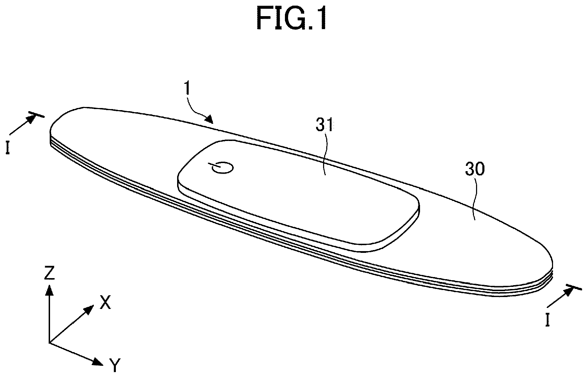

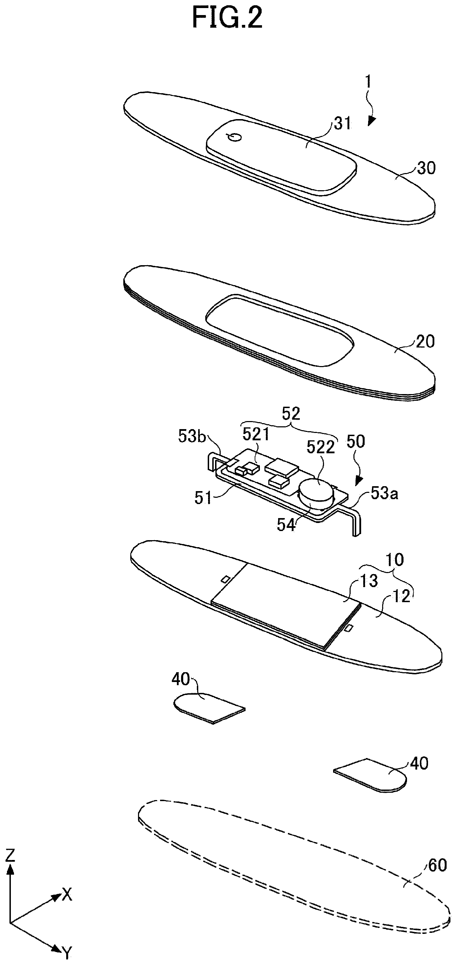

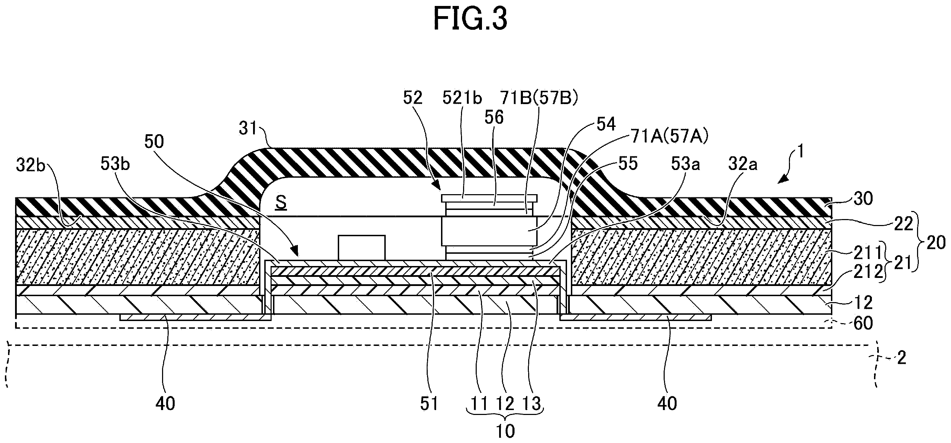

FIG. 1 is a perspective view illustrating a configuration of a biosensor according to the present embodiment. FIG. 2 is an exploded perspective view of FIG. 1. FIG. 3 is a cross-sectional view of I-I in FIG. 1. As shown in FIG. 1, the biosensor 1 is a plate-like (sheet-like) member that is approximately elliptically formed in a plan view. As shown in FIGS. 2 and 3, the biosensor 1 includes a biological adhesive layer 10, a foamed sheet 20, a casing 30, an electrode 40, and a sensor portion 50, and is constituted by laminating the biological adhesive layer 10, the foamed sheet 20, and the casing 30 in this order from the biological adhesive layer 10 side toward the casing 30 side. The biological adhesive layer 10, the foamed sheet 20, and the casing 30 have substantially the same outer shape in a plan view. The electrode 40 is provided on the surface of the biological adhesive layer 10 on the side of affixing to the skin 2 (-Z-axis direction). The sensor portion 50 is disposed on the biological adhesive layer 10 and is housed in a storage space S formed of the foamed sheet 20 and the casing 30.

### [Biological Adhesive Layer]

As shown in FIG. 3, the biological adhesive layer 10 includes a substrate 11, a sticking adhesive layer 12, and a sensor adhesive layer 13.

### (Substrate)

As shown in FIG. 3, the substrate 11 is provided on the sticking adhesive layer 12, so that a surface on the side of the foamed sheet 20 and the casing 30 is exposed. The outer shape of both sides of the substrate 11 in the width direction (X-axis direction) is substantially the same as the outer shape of both sides of the foamed sheet 20 and the casing 30 in the width direction (X-axis direction). The length (Y-axis direction) of the substrate 11 is formed shorter than the length (Y-axis direction) of the foamed sheet 20 and the casing 30.

The substrate 11 can be formed of a flexible resin having appropriate elasticity, flexibility, and toughness. As a material for forming the substrate 11, for example, a thermoplastic resin including a polyester-based resin, such as polyethylene terephthalate (PET), polybutylene terephthalate (PBT), polytrimethylene terephthalate, polyethylene naphthalate, and polybutylene naphthalate; an acrylic-based resin, such as polyacrylic acid, polymethacrylic acid, polymethyl acrylate, polymethyl methacrylate (PMMA), polyethyl methacrylate, and polybutyl acrylate; a polyolefin-based resin, such as polyethylene and polypropylene; a polystyrene-based resin, such as polystyrene, imide-modified polystyrene, acrylonitrile-butadiene styrene (ABS) resin, imide-modified ABS resin, styrene-acrylonitrile copolymerization (SAN) resin, and acrylonitrile-ethylene-propylene-diene styrene (AES) resin; a polyimide-based resin; a polyurethane-based resin; a silicone-based resin; and a polyvinyl chloride-based resin, such as polyvinyl chloride resin, and vinyl chloride-vinyl acetate copolymer resin, may be used. Among them, a polyolefin-based resin and PET are preferably used. The above-described thermoplastic resins are waterproof (with low moisture permeability). Thus, when the substrate 11 is formed of the above-described thermoplastic resins, it is possible to prevent perspiration or water vapor generated from the skin 2 from entering a flexible substrate 51 side of the sensor portion 50 through the substrate 11 in the state where the biosensor 1 is affixed to the skin 2 of the living body.

Since the sensor portion 50 is disposed on the upper surface of the substrate 11, the substrate 11 is preferably formed in a flat plate shape.

The thickness of the substrate 11 can be selected appropriately and optionally, and is for example, preferably 1 um - 300 um, more preferably 5 um - 100 um, and even more preferably 10 um - 50 um.

### (Sticking adhesive layer)

As shown in FIG. 3, the sticking adhesive layer 12 is provided on the lower surface of the substrate 11 on the sticking side (-Z-axis direction), and is a layer that is brought into contact with the living body. The sticking adhesive layer 12 preferably has moisture permeability. Water vapor or the like generated from the skin 2, to which the biosensor 1 is affixed, can be released to the foamed sheet 20 through the sticking adhesive layer 12. Further, since the foamed sheet 20 has a bubble structure as will be described later, water vapor can be discharged to the outside of the biosensor 1 through a casing adhesive layer 22. Thus, it is possible to prevent perspiration or water vapor from accumulating at the interface between the skin 2 on which the biosensor 1 is mounted and the sticking adhesive layer 12. As a result, it is possible to prevent the adhesive force of the sticking adhesive layer 12 from being weakened by the moisture accumulated at the interface between the skin 2 and the sticking adhesive layer 12, and peeling of the biosensor 1 from the skin can be suppressed.

The sticking adhesive layer 12 preferably has pressure sensitive adhesiveness. Since the sticking adhesive layer 12 has pressure sensitive adhesiveness, the biosensor 1 can be easily affixed to the skin 2 by pressing the biosensor 1 against the skin 2 of the living body.

The material of the sticking adhesive layer 12 is not particularly limited, as long as the material has a pressure-sensitive adhesiveness. The material includes a biocompatible material, and the like. Suitable materials forming the sticking adhesive layer 12 include, for example, an acrylic pressure-sensitive adhesive, and a silicone pressure-sensitive adhesive. The material preferably includes an acrylic pressure-sensitive adhesive.

The acrylic pressure-sensitive adhesive preferably includes an acrylic polymer as a main component. The acrylic polymer can function as a pressure sensitive adhesive component. For the acrylic polymer, a polymer obtained by polymerizing monomer components including a (meth)acrylic ester, such as isononyl acrylate or methoxyethyl acrylate, as a main component, and a monomer that can be copolymerized with (meth)acrylic ester, such as acrylic acid, as an optional component, may be used.

Preferably, the acrylic pressure-sensitive adhesive further includes a carboxylic acid ester. The carboxylic acid ester functions as a pressure-sensitive adhesive force regulator to reduce a pressure-sensitive adhesive force of the acrylic polymer to adjust the pressure-sensitive adhesive force of the sticking adhesive layer 12. For the carboxylic acid ester, a carboxylic acid ester compatible with acrylic polymers may be used. For the carboxylic acid ester, a trifatty acid glyceryl or the like may be used.

The acrylic pressure-sensitive adhesive may contain a crosslinking agent, as necessary. The crosslinking agents are cross-linking components that cross-link acrylic polymers. Suitable crosslinking agents include, for example, a polyisocyanate compound (a polyfunctional isocyanate compound), an epoxy compound, a melamine compound, a peroxide compound, a urea compound, a metal alkoxide compound, a metal chelate compound, a metal salt compound, a carbodiimide compound, an oxazoline compound, an aziridine compound, and an amine compound. Among the above-described compounds, the polyisocyanate compound is preferable. The above-described crosslinking agents may be used singly, or a combination of two or more crosslinking agents may be used.

The sticking adhesive layer 12 preferably has excellent biocompatibility. For example, when the sticking adhesive layer 12 is subjected to a keratin peeling test, a keratin peeling area ratio is preferably 0% - 50%, and more preferably 1% - 15%. When the keratin peeling area ratio is 0% - 50%, the load on the skin 2 can be suppressed even when the sticking adhesive layer 12 is affixed to the skin 2.

The moisture permeability of the sticking adhesive layer 12 is preferably 300 g/m²·day or more, more preferably 600 g/m²·day or more, and even more preferably 1,000 g/m²·day or more. Moreover, the moisture permeability of the sticking adhesive layer 12 is 10,000 g/m²·day or less. If the moisture permeability of the sticking adhesive layer 12 is 300 g/m²·day or more, perspiration or the like generated from the skin 2 can be transmitted appropriately from the substrate 11 to the outside even when the sticking adhesive layer 12 is affixed to the skin 2, thereby the load to the skin 2 can be reduced.

The thickness of the sticking adhesive layer 12 can be appropriately selected. The thickness is preferably 10 um - 300 um. When the thickness of the sticking adhesive layer 12 is 10 um - 300 um, the biosensor 1 can be made thinner.

The sensor adhesive layer 13 is stuck to the upper surface of the substrate 11 and is a layer for bonding the substrate 11 to the flexible substrate 51 of the sensor portion 50. The material for forming the sensor adhesive layer 13 is preferably a material having pressure-sensitive adhesiveness, the same material as the sticking adhesive layer 12 can be used, and an acrylic pressure-sensitive adhesive is preferably used.

The thickness of the sensor adhesive layer 13 can be set optionally and appropriately, and is preferably 10 um - 300 um. When the thickness of the sensor adhesive layer 13 is 10 um - 300 um, the biosensor 1 can be made thinner.

### [Foamed Sheet]

The foamed sheet 20 has a foamed sticking layer 21 and a casing adhesive layer 22 provided on the upper surface thereof.

### (Foamed sticking layer)

As shown in FIG. 3, the foamed sticking layer 21 includes a foamed substrate 211 and a substrate adhesive layer 212 provided on the surface of the foamed substrate 211 on the living body side (-Z-axis direction). When the foamed substrate 211 and the sticking adhesive layer 12 are sufficiently bonded, the foamed sticking layer 21 may not include the substrate adhesive layer 212, and the foamed substrate 211 may be bonded to the sticking adhesive layer 12.

### ((Foamed Substrate))

The foamed substrate 211 can be formed using a foam having a foaming structure of open cell, closed cell, or semi-closed cell having flexibility, waterproofness, and moisture permeability. Thereby, water vapor due to perspiration or the like generated from the living body to which the biosensor 1 is affixed can be discharged to the outside of the biosensor 1 through the foamed substrate 211.

As the material for forming the foamed substrate 211, for example, a thermoplastic resin such as a polyurethane resin, a polystyrene resin, a polyolefin resin, a silicone resin, an acrylic resin, a vinyl chloride resin, or a polyester resin may be used. Among the above-described resins, a polyester resin is preferable, and PET is suitably used.

The thickness of the foamed substrate 211 can be suitably selected, and can be, for example, 0.5 mm - 1.5 mm.

### ((Substrate Adhesive Layer))

As shown in FIG. 3, the substrate adhesive layer 212 is stuck to the lower surface of the foamed substrate 211 and has a function of bonding the substrate 11 to the foamed substrate 211.

The substrate adhesive layer 212 preferably has moisture permeability. Water vapor or the like generated from the skin 2 to which the biosensor 1 is affixed can be released to the foamed substrate 211 through the substrate adhesive layer 212. Further, since the foamed substrate 211 has a bubble structure as described above, water vapor can be discharged to the outside of the biosensor 1 through the casing adhesive layer 22. Thus, it is possible to prevent perspiration or water vapor from accumulating at the interface between the skin 2 on which the biosensor 1 is mounted and the sticking adhesive layer 12. As a result, it is possible to prevent the adhesive force of the sticking adhesive layer 12 from being weakened by the moisture accumulated at the interface between the skin 2 and the sticking adhesive layer 12, and the biosensor 1 can be prevented from peeling off from the skin 2.

The material for forming the substrate adhesive layer 212 is preferably a material having pressure-sensitive adhesiveness, and the same material as that of the sticking adhesive layer 12 can be used. As the material for forming the substrate adhesive layer 212, an acrylic pressure-sensitive adhesive is preferably used.

The moisture permeability of the substrate adhesive layer 212 is preferably 1 g/m²·day or more, more preferably 10 g/m²·day or more. The moisture permeability of the substrate adhesive layer 212 is 10,000 g/m²·day or less. When the moisture permeability of the substrate adhesive layer 212 is 10 g/m²·day or more, when the sticking adhesive layer 12 is affixed to the skin 2, perspiration or the like transmitted from the biological adhesive layer 10 can be permeated to the outside, so that the load on the skin 2 can be suppressed.

The thickness of the substrate adhesive layer 212 can be set appropriately and as desired, and is preferably 10 um - 300 um. When the thickness of the substrate adhesive layer 212 is 10 um - 300 um, the thickness of the biosensor 1 can be reduced.

### (Casing Adhesive Layer)

As shown in FIG. 3, the casing adhesive layer 22 is provided in a state of being stuck to the upper surface of the foamed substrate 211. The casing adhesive layer 22 is stuck at a position corresponding to a flat surface on the sticking side (-Y-axis direction) of the casing 30 on the upper surface of the foamed substrate 211, and has a function of bonding the foamed substrate 211 to the casing 30.

As the material for forming the casing adhesive layer 22, a silicon-based adhesive, a silicon tape or the like can be used.

The casing adhesive layer 22 preferably has moisture permeability. Since the foamed substrate 211 has an open cell structure, water vapor entering through the casing adhesive layer 22 can be discharged to the outside of the biosensor 1.

The thickness of the casing adhesive layer 22 can be set appropriately, and is, for example, 0.5 mm - 1.5 mm.

The humidity permeability of the casing adhesive layer 22 is preferably 65 g/m²·day - 4,000 g/m²·day, more preferably 90 g/m²·day - 2,000 g/m²·day, even more preferably 800 g/m²·day - 1,700 g/m²·day, and most preferably 850 g/m²·day - 1,660 g/m²·day. If the moisture permeability of the casing adhesive layer 22 is within the above preferred range, the stress at the interface between the skin 2 and the sticking adhesive layer 12 provided on the surface of the substrate 11 on the sticking side (-Z-axis direction) can be relaxed, so that the peeling of the biosensor 1 from the skin 2 can be suppressed.

### (Casing)

As shown in FIG. 3, the casing 30 is stuck to the upper surface of the foamed sheet 20. The casing 30 has a substantially dome-shaped projection portion 31 protruding in the height direction (+Z-axis direction) of the biosensor 1 at the center in the longitudinal direction (Y-axis direction). The lower surface of the casing 30 (the surface on the sticking side) is formed flat. A storage space S for storing the sensor portion 50 is formed inside (on the sticking side) of the projection portion 31.

As the material for forming the casing 30, a material having flexibility such as silicone rubber, fluororubber, or urethane rubber can be used. The material protects the sensor portion 50 arranged in the storage space S of the casing 30, and absorbs an impact applied to the biosensor 1 from the upper surface side, to reduce the impact applied to the sensor portion 50.

The thickness of the top side and the side wall of the projection portion 31 of the casing 30 is preferably greater than the thickness of the flat portions 32a and 32b provided on both ends in the longitudinal direction L of the casing 30. Thus, the flexibility of the projection portion 31 can be made lower than that of the flat portions 32a and 32b, and the sensor portion 50 can be protected from an external force applied to the biosensor 1.

Preferably, the thickness of the top side and side wall of the projection portion 31 is 1.5 mm - 3 mm, and the thickness of the flat portions 32a and 32b is 0.5 mm - 1 mm.

Since the thin flat portions 32a and 32b are more flexible than the projection portion 31, when the biosensor 1 is affixed to the skin 2, the flat portions can be easily deformed following deformation of the surface of the skin 2 caused by body movement such as stretching, bending and twisting. Thus, the stress applied to the flat portions 32a and 32b when the surface of the skin 2 is deformed can be relaxed, and the biosensor 1 can be made unlikely to peel off from the skin 2.

The outer peripheral portions of the flat portions 32a and 32b preferably have a shape in which the thickness gradually decreases toward the end. Thus, the flexibility of the outer peripheral portions of the flat portions 32a and 32b can be further enhanced, and the wearing feeling when the biosensor 1 is affixed to the skin 2 can be improved as compared with the case where the thickness of the outer peripheral portions of the flat portions 32a and 32b is not reduced.

### (Electrode)

As shown in FIG. 3, the electrode 40 is stuck to the lower surface of the sticking adhesive layer 12 provided on the lower surface of the substrate 11 on the sticking side (-Z-axis direction). When the biosensor 1 is affixed to the skin 2, the electrode 40 is brought into contact with the skin 2, so that a biological signal can be detected. The biological signal is, for example, an electric signal representing an electrocardiogram, an electroencephalogram, or a pulse. Note that the electrode 40 may be embedded in the substrate 11 in a state of being exposed contactably with the skin.

The electrode 40 can be formed by using an electrode sheet in which a cured product of a conductive composition containing a conductive polymer and a binder resin, a metal, an alloy, or the like is formed in a sheet shape.

For the conductive polymer, for example, a polythiophene-based conductive polymer, a polyaniline-based conductive polymer, a polypyrrole-based conductive polymer, a polyacetylene-based conductive polymer, a polyphenylene-based conductive polymer and derivatives thereof, and a complex thereof may be used. The above-described conductive polymers may be used singly, or a combination of two or more conductive polymers may be used. Among them, a complex obtained by doping polyaniline as a dopant to polythiophene is preferably used. Among the complexes of polythiophene and polyaniline, PEDOT/PSS obtained by doping polystyrene sulfonic acid (poly4-styrene sulfonate; PSS) to poly3,4-ethylene dioxythiophene (PEDOT), is more preferably used because of a lower contact impedance with the living body and the high electric conductivity.

Further, the electrode 40 may be embedded in the lower surface of the sticking adhesive layer 12 in a state of being exposed to the sticking adhesive layer 12 contactably with the living body.

The electrode 40 may have a plurality of through holes on the contact surface with the skin 2. Thereby, the electrode 40 can expose the sticking adhesive layer 12 to the sticking side from the through holes in the state of being stuck to the sticking adhesive layer 12, so that adhesiveness of the electrode 40 with the skin 2 can be enhanced.

### (Sensor portion)

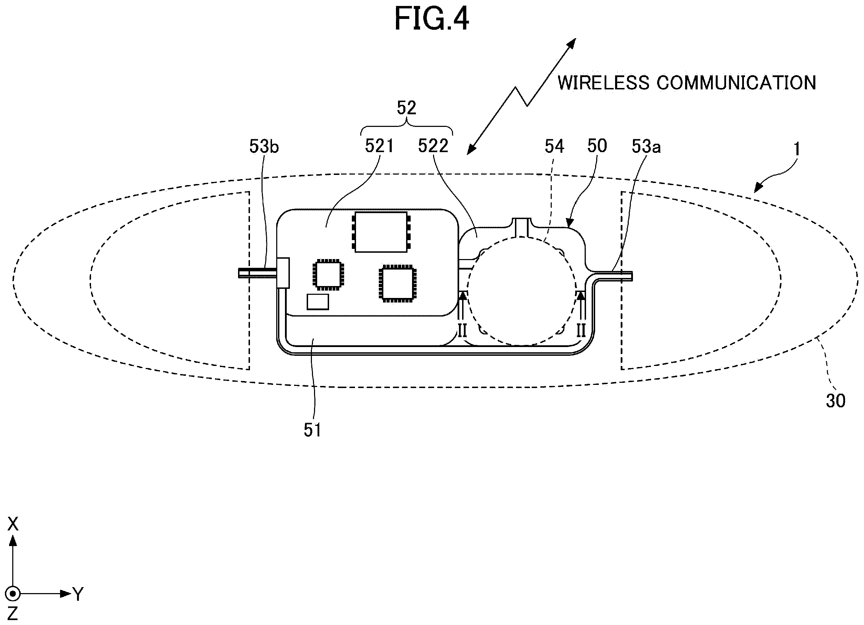

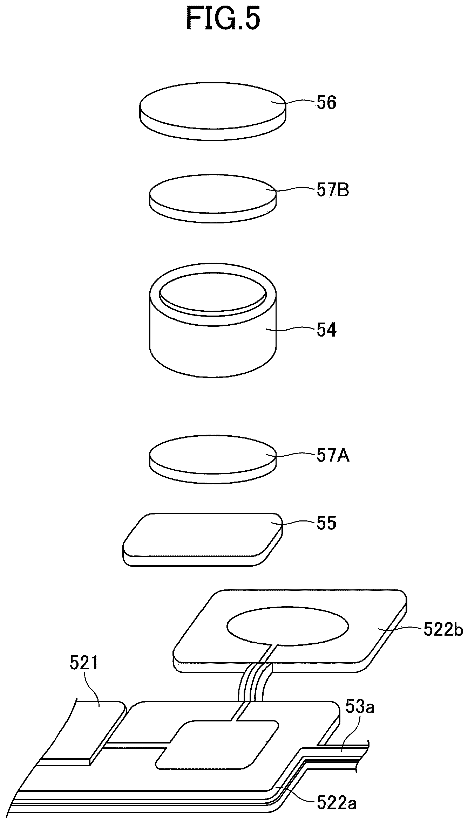

FIG. 4 is a plan view illustrating a configuration of the sensor portion 50, and FIG. 5 is an exploded perspective view of a part of the sensor portion 50. The dashed line in FIG. 4 represents the outer shape of the casing 30. As shown in FIGS. 4 and 5, the sensor portion 50 includes a flexible substrate 51 on which various components for acquiring biological information are mounted, a sensor body 52, wirings 53a and 53b connected to the sensor body 52 in the longitudinal direction of the sensor body 52, a battery 54, a positive electrode pattern (first electrode portion) 55, a negative electrode pattern (second electrode portion) 56, and a conductive adhesive tape 57. Between a pad portion 522a and a pad portion 522b of the sensor portion 50, the positive electrode pattern 55, the conductive adhesive tape 57, the battery 54, the conductive adhesive tape 57, and the negative electrode pattern 56 are laminated in this order from the pad portion 522a side to the pad portion 522b side. In the present embodiment, the positive terminal of the battery 54 is set to be in the -Z-axis direction and the negative terminal is set to be in the +Z-axis direction. However, the positive terminal and the negative terminal may be reversed, i.e. the positive terminal may be in the +Z-axis direction and the negative terminal may be in the -Z-axis direction.

The flexible substrate 51 is made of a resin, and the flexible substrate 51 is integrally formed with the sensor body 52 and the wirings 53a and 53b.

An end of each of the wirings 53a and 53b is connected to the electrode 40, as shown in FIG. 3. As shown in FIG. 4, the other end of the wiring 53a is connected to a switch or the like mounted to a component mounting portion 521 along the outer periphery of the sensor body 52. The other end of the wiring 53b is connected to a switch or the like mounted on the component mounting portion 521 in the same manner as the wiring 53a. The wirings 53a and 53b may be formed on any of wiring layers on the front surface side and the rear surface side of the flexible substrate 51.

As shown in FIG. 4, the sensor body 52 includes the component mounting portion 521 that is a controller and includes the battery mounting portion 522.

The component mounting portion 521 includes various components mounted on the flexible substrate 51, such as a CPU and an integrated circuit for processing biological signals acquired from a living body to generate biological signal data; a switch for activating the biosensor 1; a flash memory for storing the biological signals; or a light emitting element. Examples of circuits using various components will be omitted. The component mounting portion 521 is operated by power supplied from the battery 54 mounted on the battery mounting portion 522.

The component mounting portion 521 by wire or wirelessly communicates with an external device such as an operation checking device for checking an initial operation, or a readout device for reading biological information from the biosensor 1.

The battery mounting portion 522 supplies power to the integrated circuit mounted on the component mounting portion 521. The battery 54 is mounted on the battery mounting portion 522, as shown in FIG. 5.

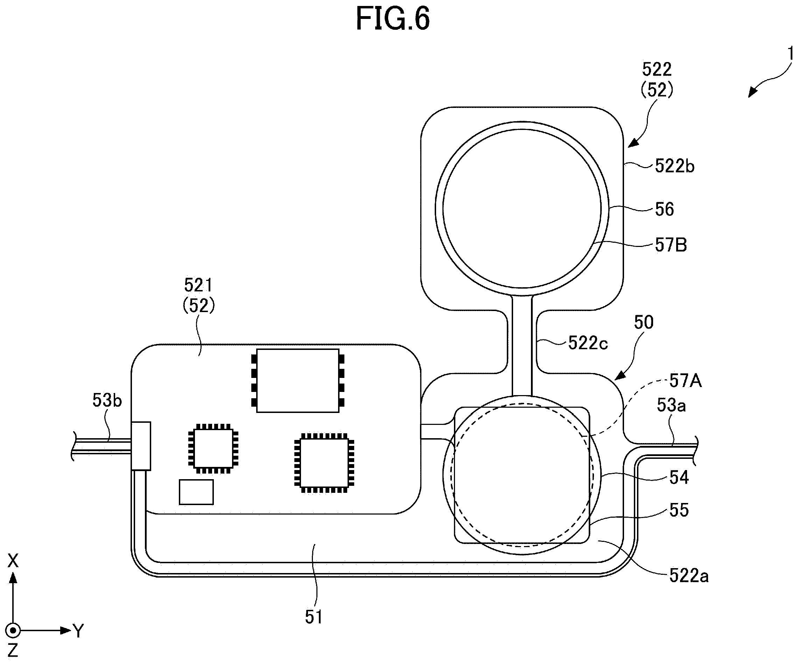

FIG. 6 is an explanatory diagram showing a layout of the sensor portion 50 shown in FIG. 4. As shown in FIG. 6, the battery mounting portion 522 is disposed between the wiring 53a and the component mounting portion 521, and includes the pad portions 522a and 522b and a constriction portion 522c.

As shown in FIG. 6, the pad portion 522a is provided between the wiring 53a and the component mounting portion 521, located on the positive terminal side of the battery 54, and has the positive electrode pattern 55 to which the positive terminal is connected.

As shown in FIG. 6, the pad portion 522b is provided separated from the pad portion 522a by a predetermined distance along a direction orthogonal to the longitudinal direction (in the vertical direction in FIG. 3) with respect to the pad portion 522a. The pad portion 522b is located on the negative terminal (second terminal) side of the battery 54 and has the negative electrode pattern 56 to which the negative terminal is connected.

As shown in FIG. 6, the constriction portion 522c is disposed between the pad portions 522a and 522b to connect the pad portions 522a and 522b to each other.

As shown in FIG. 5, the battery 54 is arranged between the positive and negative electrode patterns 55 and 56. The battery 54 has positive and negative terminals. For the battery 54, a publicly-known battery may be used. The battery 54 may be a coin-type battery, such as CR2025.

The positive electrode pattern 55 is located on the positive terminal side of the battery 54 and is connected to the positive terminal. As shown in FIG. 6, the positive electrode pattern 55 has a rectangular shape with chamfered corners.

The negative electrode pattern 56 is located on the negative terminal side of the battery 54 and is connected to the negative terminal. As shown in FIG. 6, the negative electrode pattern 56 has a shape substantially corresponding to the size of the circular shape of the negative terminal of the battery 54. The diameter of the negative electrode pattern 56 is, for example, equal to the diameter of the battery 54 and approximately equal to the diagonal length of the positive electrode pattern 55.

The conductive adhesive tape 57 is a conductive adhesive that is disposed between the battery 54 and the positive electrode pattern 55 and also is disposed between the battery 54 and the negative electrode pattern 56. The conductive adhesive tape may also generally be referred to as a conductive adhesive sheet, a conductive adhesive film, or the like.

In the conductive adhesive tape 57, when an iron ball having a weight of 33 g is dropped vertically from a height of 30 cm to apply a load to the surface, the fluctuation width of the resistance value is set to be 1.60 Ω or less in absolute value. The inventor of the present application has focused on that by suppressing the fluctuation of the resistance value when a predetermined load is applied to the surface of the conductive adhesive tape 57, a change in a size of a conductive path can be suppressed even if a load is applied to the conductive adhesive tape 57. The present inventor has found that when the fluctuation width of the resistance value of the conductive adhesive tape 57 is 1.60 Ω or less in absolute value, the change in the size of the conductive path can be suppressed even if the predetermined load is applied to the conductive adhesive tape 57, and a stable conduction can be secured.

The conductive adhesive tape 57 preferably has a bending load per unit bending deflection of 0.035 N/cm or less. When the rigidity of the conductive adhesive tape 57 for joining the battery to the positive electrode pattern 55 or to the negative electrode pattern 56 is reduced and the bending load per unit bending deflection is a predetermined value or less, a change in a size of a conductive path can be suppressed even if a load is applied to the conductive adhesive tape 57. , resistance fluctuation of the conductive adhesive tape 57 is suppressed and a stable conduction can be secured.

Here, the bending load per unit bending deflection can be obtained by calculating a bending load per unit bending deflection using the three-point bending test in accordance with JIS K7171. Specifically, a conductive adhesive tape 57 having a predetermined size (for example, 2 cm in width × 15 cm in length) is prepared. The conductive adhesive tape 57 is reinforced by sandwiching with PET films (thickness: 38 um) from both sides. Thus, a laminate in which the conductive adhesive tape 57 is sandwiched by the PET film is produced. The three-point bending test is performed using the laminate, and the deflection up to 1 cm of a deflection - load curve with the deflection (unit: cm) in the X-axis and the load (unit: N) in the Y-axis is regarded as an initial inclination, and the inclination of the laminate is calculated. The obtained inclination of the laminate is obtained as a bending load per unit bending deflection of the conductive adhesive tape 57. When a plurality of laminates (for example, 9) are produced, the bending load per unit bending deflection may be an average value of bending loads per unit bending deflection of the plurality of laminates (for example, 9). It should be noted that the distance between the two fulcrums where the conductive adhesive tape 57 is to be installed and the lowering (compression) speed of the indenter can be appropriately selected in accordance with the size of the laminate or the like, for example, when the size of the conductive adhesive tape 57 is 2 cm in width × 15 cm in length, the distance between the fulcrums is set to 5 cm and the compression speed is set to 1 cm/min.

The conductive adhesive tape 57 includes a first conductive adhesive tape 57A disposed between the positive terminal of the battery 54 and the positive electrode pattern 55, and a second conductive adhesive tape 57B disposed between the negative terminal of the battery 54 and the negative electrode pattern 56. In the following description, the first conductive adhesive tape 57A and the second conductive adhesive tape 57B may be collectively referred to simply as the conductive adhesive tape 57.

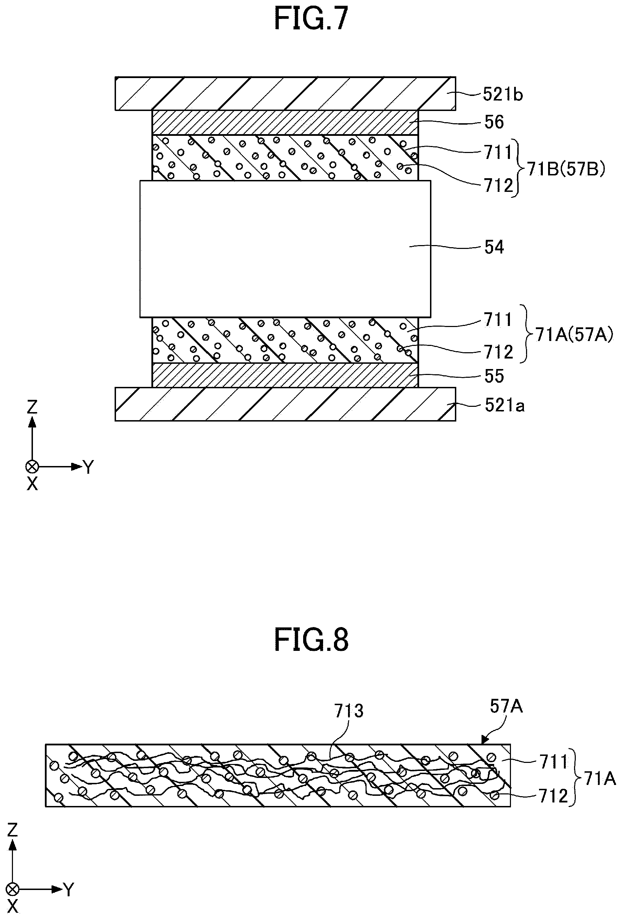

FIG. 7 is a partial cross-sectional view of II-II in FIG. 4. As shown in FIG. 7, the first conductive adhesive tape 57A is provided between the positive terminal of the battery 54 and the positive electrode pattern 55, and electrically connects the positive terminal and the positive electrode pattern 55. The second conductive adhesive tape 57B is provided between the negative terminal of the battery 54 and the negative electrode pattern 56, and electrically connects the second terminal and the negative electrode pattern 56.

The first conductive adhesive tape 57A is composed of an adhesive layer 71A, and the second conductive adhesive tape 57B is composed of an adhesive layer 71B. Both the first conductive adhesive tape 57A and the second conductive adhesive tape 57B are so-called double-sided adhesive type conductive adhesive tapes in which both surfaces of the adhesive layers 71A and 71B are adhesive surfaces. The surfaces of the adhesive layers 71A and 71B may be referred to as adhesive surfaces. The first conductive adhesive tape 57A and the second conductive adhesive tape 57B are so-called substrate-less conductive double-sided adhesive tapes which are not provided with a conductive substrate such as a metal foil.

### [Adhesive Layer]

The adhesive layers 71A and 71B have conductivity (electric conductivity) while providing adhesive surfaces of the conductive adhesive tapes. When the adhesive surfaces of the adhesive layers 71A and 71B are adhered to an adherend such as a conductor, electric conduction between the adherend and the adhesive layers 71A and 71B is ensured.

As shown in FIG. 7, the adhesive layers 71A and 71B include an adhesive resin 711 and conductive fine particles (conductive fillers) 712. The adhesive layers 71A and 71B may contain other components (additives) to the extent that the purpose of the conductive adhesive tape 57 is not impaired.

The thickness of the adhesive layers 71A and 71B is preferably 20 um - 80 um, more preferably 30 um - 70 um, and even more preferably 35 um - 60 um. When the thickness of the adhesive layers 71A and 71B is within the above preferred range, the conductive adhesive tape 57 can be made thin, so that flexibility can be enhanced.

### (Adhesive Resin)

The adhesive resin 711 has a function of securing the adhesive force of the adhesive layers 71A and 71B. The adhesive resin 711 is formed using an adhesive polymer. Examples of the adhesive polymer include an acrylic polymer, a silicone polymer, a urethane polymer, a rubber polymer, a vinyl alkyl ether polymer, a polyester polymer, a polyamide polymer, a fluorine polymer, and an epoxy polymer. Among the above-described polymers, an acrylic polymer is preferably used in view of ease of designing the polymer, ease of adjusting the adhesive force, and ensuring dispersibility of the conductive particles. The above-described adhesive polymers may be used singly, or a combination of two or more adhesive polymers may be used. In the following description, a case where the adhesive polymer is composed of an acrylic polymer or contains an acrylic polymer will be described.

The content (lower limit value) of the adhesive resin 711, with respect to the total mass (100 mass%) of the adhesive layers 71A and 71B, is preferably 20 mass% or more, more preferably 25 mass% or more, and even more preferably 30 mass% or more. The content (upper limit value) of the adhesive resin 711, based on the total mass (100 mass%) of the adhesive layers 71A and 71B, is preferably 60 mass% or less, and more preferably 55 mass% or less.

When the adhesive resin 711 is formed containing an acrylic polymer, the content (lower limit value) of the acrylic polymer, with respect to the total mass (100 mass%) of the adhesive resin 711, is preferably 50 mass% or more, and more preferably 60 mass% or more. The content (upper limit value) of the acrylic polymer, with respect to the total mass (100 mass%) of the resin component, is preferably 100 mass% or less, and more preferably 90 mass% or less.

The acrylic polymer is not particularly limited, but is preferably an acrylic polymer composed of, for example, a (meth)acrylic acid alkyl ester having a 1 - 20 carbon number linear or branched alkyl group (Hereinafter referred to simply as (meth)acrylic acid alkyl ester) and a polar group-containing monomer, as monomer components. In this specification, the term "(meth)acrylic" refers to "acrylic", "methacrylic", or both.

The weight average molecular weight (Mw) of the acrylic polymer is preferably 300,000 - 1,000,000, and more preferably 400,000 - 800,000, in view of being able to exhibit more of the effect of the adhesive resin 711. The weight-average molecular weight (Mw) can be controlled by the type and the amount of the polymerization initiator and the chain transfer agent, the temperature and the time of the polymerization, the monomer concentration, the monomer dropping rate, or the like. The weight-average molecular weight can be measured by, for example, Gel Permeation Chromatography (GPC).

### [Conductive Particles]

As shown in FIG. 7, the conductive fine particles 712 are contained in the adhesive resin 711 in a dispersed state.

As the conductive fine particles 712, particles having conductivity such as metal powder are used. The materials for forming the conductive particles include, for example, metals such as nickel, iron, chromium, cobalt, aluminum, antimony, molybdenum, copper, silver, platinum, or gold, alloys or oxides thereof, or particles (powder) made of a conductive material such as a carbon material, e.g., carbon black; coated particles in which surfaces of particles, such as polymer beads, glass and ceramics, are coated with a conductive material; or the like. The coated particles also include particles in which surfaces of particles made of a conductive material are coated with another type of conductive material. Among these particles, metals and coated particles are preferable, and nickel and silver are preferably used as the metals.

The shape of the conductive fine particles 712 may be spherical, spiky (chestnut), flaky (thin piece), filamentous, or the like, and may be selected as appropriate. When the conductive fine particles 712 are spherical, the conductive fine particles 712 are easily dispersed uniformly in the adhesive resin 711, so that the adhesive force is easily secured and the conductive path by the conductive fine particles 712 in the adhesive layer 71 is easily formed. Therefore, the shape of the conductive fine particles 712 is preferably spherical.

The aspect ratio of the conductive fine particles 712 is preferably 1.0 - 1.5, and more preferably 1.0 - 1.1. The aspect ratio of the conductive fine particles 712 can be measured by, for example, a scanning electron microscope (SEM).

The particle diameter d₅₀ of the conductive fine particles 712 is preferably smaller than the thickness of the adhesive layers 71A and 71B. That is, the relation "the thickness of the adhesive layers 71A and 71B > d₅₀" is preferably satisfied. d₅₀ is a 50% cumulative value (median diameter) in the particle size distribution. d₅₀ is measured by, for example, a laser diffraction/scattering method. When two or more kinds of conductive fine particles 712 are contained in the adhesive layers 71A and 71B, d₅₀ is calculated from the distribution obtained by mixing all kinds of conductive fine particles 712.

The above relationship of d₅₀ allows the adhesive layers 71A and 71B to have higher conductivity and more excellent adhesion. When d₅₀ is greater than or equal to the thickness of the adhesive layer 71, more than half of the conductive fine particles 712 are larger than the thickness of the adhesive layers 71A and 71B, and protrusions are formed on the surfaces of the adhesive layers 71A and 71B. Therefore, the contact area between the adhesive layers 71A and 71B and the adherend may be reduced, resulting in a decrease in adhesiveness, and the appearance may be poor. The specific range of d₅₀ is preferably 2 um - 20 um, more preferably 3 um - 15 um, and even more preferably 4 um - 10 um.

The upper limit value of the content of the conductive fine particles 712, with respect to the total mass (100 mass%) of the adhesive layers 71A and 71B, is preferably 70 mass% or less, more preferably 65 mass% or less, and even more preferably 60 mass% or less. The lower limit value of the content of the conductive fine particles 712 is preferably 15 mass% or more, more preferably 20 mass% or more, and even more preferably 25 mass% or more. When the content of the conductive fine particles 712 is within the above preferred range, the conductivity of the adhesive layers 71A and 71B can be ensured without lowering the adhesive strength of the adhesive layers 71A and 71B.

The adhesive layers 71A and 71B may include conductive fibers 713 as conductive fillers, as shown in FIG. 8.

The conductive fiber 713 may be any fiber having conductivity, and a publicly known fiber may be used. As the conductive fibers 713, metal fibers such as gold, silver, platinum, copper, nickel, tin, zinc, palladium, indium tin oxide, or copper sulfide; carbon fibers such as carbon nanotubes; fibers composed of conductive polymers; fibers composed of polymers in which fibrous or granular conductive fillers are dispersed; or conductive coated fibers in which a surface of a fiber base material is coated with a conductive material, can be used. As the fiber base material, publicly known fibers can be used regardless of presence or absence of conductivity, for example, synthetic fibers such as polyester fibers, nylon fibers, acrylic fibers, polyethylene fibers, polypropylene fibers, vinyl chloride fibers, aramid fibers, polysulfone fibers, polyether fibers, or polyurethane fibers; natural fibers such as cotton, hemp, or silk; semi-synthetic fibers such as acetate; or regenerated fibers such as rayon or cupra, can be used. The above-described fibers may be used singly, or a combination of two or more fibers may be used. Examples of the conductive material for coating the surface of the fiber base material include a metal, and a conductive polymer. As the metal, a metal similar to the metal used for the metal fiber can be used. Among them, metal is preferable from the viewpoint of conductivity, durability, flexibility, and the like. Examples of the method for coating the metal include vapor deposition, sputtering, electroplating, electroless plating, and the like.

The fiber diameter of the conductive fiber 713 is preferably less than 1.1 um, more preferably 300 nm - 1.0 um, even more preferably 400 nm - 700 nm. If the content of the conductive fibers 713 is within the above preferred range, the conductivity of the adhesive layer 71 can be ensured without lowering the adhesive force of the adhesive layers 71A and 71B.

The fiber length of the conductive fiber 713 is not particularly limited, and can be appropriately selected according to the type, thickness, volume, and the like of the adhesive layers 71A and 71B. The fiber diameter and the fiber length of the conductive fiber 713 can be measured by a scanning electron microscope.

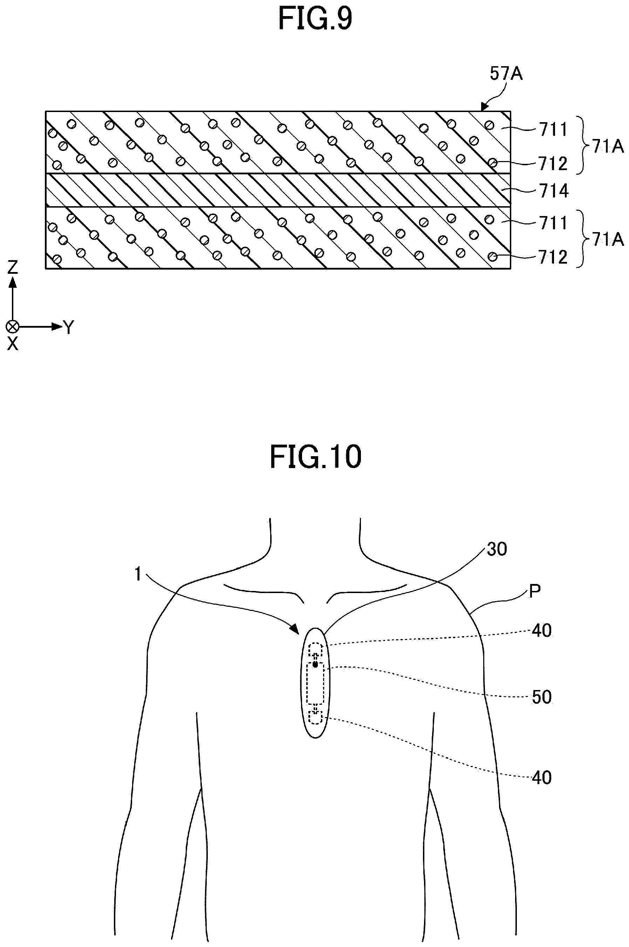

The adhesive layers 71A and 71B may include a conductive substrate as shown in FIG. 9. A conductive double-sided adhesive tape provided with a conductive substrate in the adhesive layers 71A and 71B is referred to as a so-called conductive double-sided adhesive tape with a substrate. That is, the conductive adhesive tape 57 may be constituted by providing the adhesive layers 71 on both surfaces of the conductive substrate 714.

Further, the adhesive layer 71 may include other layers such as an intermediate layer, or an undercoat layer, in addition to the conductive fibers and the conductive substrate, to the extent that the purpose of the conductive adhesive tape 57 is not impaired.

When the battery 54 is attached to the biosensor 1, the first conductive adhesive tape 57A and the second conductive adhesive tape 57B are attached to the entire positive electrode pattern 55 and the entire negative electrode pattern 56, respectively. Then, the positive terminal and the negative terminal of the battery 54 are respectively stuck to the positive electrode pattern 55 and the negative electrode pattern 56 via the first conductive adhesive tape 57A and the second conductive adhesive tape 57B, so that the battery 54 is mounted on the battery mounting portion 522. The sensor body 52 shown in FIG. 4 shows a state in which the battery 54 is sandwiched between the positive electrode pattern 55 and the negative electrode pattern 56 with the constriction portion 522c being bent, so that the battery is mounted on the battery mounting portion 522.

As shown in FIG. 3, for the biosensor 1, in order to protect the biological adhesive layer 10 and the electrode 40 on the sticking surface side (-Z-axis direction), a release paper 60 is preferably stuck until the biosensor 1 is affixed to the living body. The adhesive force of the sticking adhesive layer 12 of the biological adhesive layer 10 can be maintained by peeling the release paper 60 from the biological adhesive layer 10 and the electrode 40 when the biosensor 1 is used.

FIG. 10 is an explanatory diagram showing a state in which the biosensor 1 of FIG. 1 is affixed to a chest of a living body P. For example, the biosensor 1 is affixed to a skin of a subject P, the longitudinal direction (Y-axis direction) aligned with the sternum of the subject P, with one electrode 40 on the upper side and the other electrode 40 on the lower side. The biosensor 1 acquires a biological signal such as an electrocardiogram signal from the subject P by means of the electrode 40 in a state where the electrode 40 is pressed to the skin of the subject P by sticking the sticking adhesive layer 12 in FIG. 3 to the skin of the subject P. The biosensor 1 stores the acquired biological signal data in a nonvolatile memory such as a flash memory mounted on the component mounting portion 521.

As described above, the biosensor 1 includes the positive electrode pattern 55 and the negative electrode pattern 56, and in the conductive adhesive tape 57, when an iron ball having a weight of 33 g is dropped vertically from a height of 30 cm to apply a load to the surface, the fluctuation width of the resistance value is set to be 1.60 Ω or less in absolute value. Thus, by suppressing the fluctuation of the resistance value when a load is applied to the surface of the conductive adhesive tape 57, a stable conduction can be secured. Therefore, the biosensor 1 can suppress a resistance fluctuation of the conductive adhesive tape 57 and an increase in the resistance even when the biosensor 1 is pressed against the skin 2 of the subject or the subject moves during the use of the biosensor 1, thereby suppressing a decrease in the supply voltage from the battery 54 to the component mounting portion 521. Therefore, the biosensor 1 can stably supply a voltage from the battery 54 to the component mounting portion 521 by suppressing the decrease in the supply voltage from the battery 54 to the component mounting portion 521.

Therefore, the biosensor 1 can prevent the operation of the component mounting portion 521 from stopping even when the biosensor 1 is pressed against the skin of the subject or the subject moves during the use of the biosensor 1, so that biological information can be stably measured from the skin 2.

The biosensor 1 may include a positive electrode pattern 55 and a negative electrode pattern 56 as electrode portions in the sensor portion 50, and a first conductive adhesive tape 57A and a second conductive adhesive tape 57B as the conductive adhesive tape 57. When the positive terminal and the negative terminal of the battery 54 are provided in mutually opposite directions, the first conductive adhesive tape 57A can be disposed between the positive terminal of the battery 54 and the positive electrode pattern 55, and the second conductive adhesive tape 57B can be disposed between the negative terminal of the battery 54 and the negative electrode pattern 56, in the biosensor 1. Thus, the biosensor 1 can form a laminate in which the positive electrode pattern 55, the first conductive adhesive tape 57A, the battery 54, the second conductive adhesive tape 57B, and the negative electrode pattern 56 are laminated in this order between the pad portion 522a and the pad portion 522b of the sensor portion 50. Therefore, when the battery 54 is a battery in which the positive terminal and the negative terminal are arranged in opposite directions to each other, such as a button type battery, the biosensor 1 can compactly store the sensor portion 50 in the biosensor 1, and can suppress a resistance fluctuation of the conductive adhesive tape 57 and an increase in the resistance of the conductive adhesive tape 57 even when a load is applied to the inside of the casing due to pressure against the skin of the subject or body movement during use.

Further, in the biosensor 1, the conductive adhesive tape 57 may be provided with an adhesive layer 71 containing an adhesive resin 711 and conductive fine particles 712. Since the rigidity of the conductive adhesive tape 57 depends mainly on the adhesive layer 71, as the conductive adhesive tape 57 is constituted with the adhesive layer 71, the bending load per unit bending deflection of the conductive adhesive tape 57 can be easily reduced. Therefore, the biosensor 1 can more easily suppress a fluctuation of the resistance of the conductive adhesive tape 57 even when a load is applied to the inside of the casing due to pressure against the skin of the subject or body movement during use.

Further, in the biosensor 1, the conductive adhesive tape 57 can be constituted without including a substrate in the adhesive resin 711. Since the flexibility of the adhesive resin 711 can be easily increased, the rigidity of the conductive adhesive tape 57 can be reduced. Therefore, when a load is applied to the surface of the conductive adhesive tape 57, the conductive adhesive tape 57 is easily deformed, so that it is possible to suppress the resistance fluctuation and suppress more stably the increase in the resistance of the conductive adhesive tape 57.

The conductive adhesive tape 57 can be configured containing conductive fibers in the biosensor 1. The plurality of conductive fibers can be dispersed while the conductive fibers can easily contact each other in the adhesive resin 711. Therefore, in the biosensor 1, even if a load is applied to the surface of the conductive adhesive tape 57, the conductive adhesive tape 57 easily maintains the conductivity, and the fluctuation of the resistance can be more stably suppressed.

In the biosensor 1, an average fiber diameter of the conductive fibers contained in the conductive adhesive tape 57 is preferably less than 1.1 um. Thus, the conductivity of the adhesive layer 71 can be ensured without lowering the adhesive force of the adhesive layer 71. Therefore, the conductive adhesive tape 57 can suppress a resistance fluctuation more stably while maintaining the adhesive force and the conductivity.

In the biosensor 1, a thickness of the adhesive layer 71 contained in the conductive adhesive tape 57 can be set to 20 um - 80 um. Thus, since the conductive adhesive tape 57 can be made thin, the rigidity of the conductive adhesive tape 57 can be reduced, and it can be easily deformed. Further, the conductive adhesive tape 57 can be made thinner. Therefore, when a load is applied to the surface of the conductive adhesive tape 57, the fluctuation of the resistance can be more easily suppressed, and the conductive adhesive tape 57 can be formed thin.

As described above, since the biosensor 1 can stably supply a voltage from the battery to the controller, for example, it can be suitably used for a wearable device for health care such as a biosensor.

In the present embodiment, the fluctuation width of the resistance value of the first conductive adhesive tape 57A and the second conductive adhesive tape 57B when a load under a predetermined condition is applied may be set to 1.60 Ω or less in absolute value.

In this embodiment, the conductive adhesive tape 57 may be disposed at only one of between the positive terminal of the battery 54 and the positive electrode pattern 55 and between the negative terminal of the battery 54 and the negative electrode pattern 56, and a conductive adhesive or the like may be used for the other of the above.

### [Examples]

Embodiments will be described in more detail with reference to practical examples and comparative examples. However, the embodiments are not limited to these practical examples and comparative examples.

### <Example 1>

### [Preparation of Conductive Adhesive Tape]

A conductive adhesive tape 1 (Product name: 9707, manufactured by 3M Japan Limited) was prepared. The conductive adhesive tape 1 was an adhesive tape containing conductive fine particles (Material: Ag, Average Particle Size: 5 um, and Filling Rate: 56 wt%) dispersed in an adhesive resin, and was a substrate-less conductive adhesive tape containing no substrate.

### (Measurement of Thickness of Conductive Adhesive Tape)

The thickness of the adhesive layer was measured using a dial gauge specified in JIS B 7503. A contact surface of the dial gauge was flat and a diameter thereof was 5 mm. Using a test piece having a width of 150 mm, the thicknesses at 5 points were measured at equal intervals in the width direction with the dial gauge having a scale of 1/1000 mm, and an average value of the measured results was defined as the thickness of the adhesive layer. Since the conductive adhesive tape 1 was composed of the adhesive layer, the thickness of the adhesive layer was set to be the thickness of the conductive adhesive tape 1.

### (Measurement of bending load per unit bending deflection)

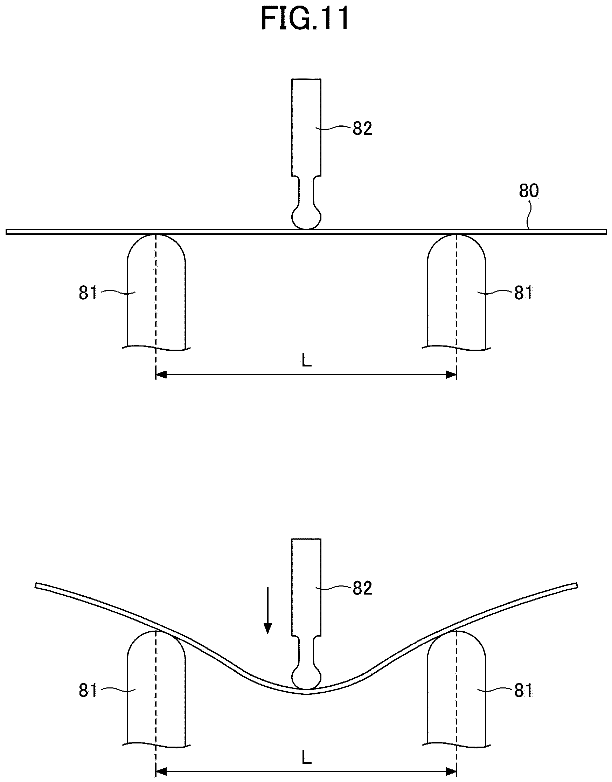

The size of the conductive adhesive tape 1 was 2 cm in width and 15 cm in length. The conductive adhesive tape 1 was reinforced by sandwiching both sides of the conductive adhesive tape 1 with a PET film (thickness: 38 pm), and 9 test pieces, in which the conductive adhesive tape 1 was sandwiched with the PET film and laminated, were prepared. The test pieces were subjected to a three-point bending test at 23°C using a small measuring device (EZ-Test, by Shimadzu Corporation) in accordance with a three-point bending test conforming to JIS K7171. An example of the three-point bending test is shown in FIG. 11. As shown in FIG. 11, a test piece 80 was placed on two fulcrums 81 so that a central portion of the test piece 80 was in the middle between the two fulcrums 81, and an indenter 82 was placed on the central portion of the test piece 80. The distance L between the fulcrums 81 was 5 cm, and the lowering (compression) speed of the indenter was 1 cm/min. The indenter was lowered to deflect the test piece 80. When the deflection (unit: cm) was on the X-axis and the load (unit: N) was on the Y-axis, the obtained deflection (X) - load (Y) curve with the deflection up to 1 cm was regarded as an initial inclination, and the inclination of the test piece 80 was calculated. The obtained inclination was defined as a bending load per unit bending deflection. The bending load per unit bending deflection was an average value of the bending loads per unit bending deflection of the 9 test pieces 80.

### (Calculation of Resistance Fluctuation Value)

The size of the test piece of the conductive adhesive tape 1 was 1 cm in width and 1 cm in length, and an iron ball weighing 33 g was dropped from a position of 30 cm directly above the conductive adhesive tape 1, with an electric current of about 50 mA flowing through the test piece. The resistance fluctuation was calculated from the voltage fluctuation when the iron ball collided with the test piece, and the peak resistance value was measured. The resistance fluctuation value was calculated from the difference between the peak resistance value and the resistance value before the ball was dropped. When the resistance fluctuation value of the conductive adhesive tape was 1.60 Ω or less, it was evaluated that the voltage stability of the conductive adhesive tape was excellent ("A" in TABLE 1), and when the resistance fluctuation value of the conductive adhesive tape exceeded 1.60 Ω, it was evaluated that the voltage stability of the conductive adhesive tape was poor ("B" in TABLE 1).

### [Preparation of Biosensor]

Both terminals of the battery and two electrode pads formed on the flexible substrate were bonded through the conductive adhesive tape 1, to prepare the sensor portion. A biological adhesive layer was prepared, in which an adhesive layer for sticking was formed on the side of the sticking surface of the substrate, and an adhesive layer was formed on the side opposite to the side of the sticking surface of the substrate. Further, a foamed sheet was prepared in which an adhesive layer for a casing is provided on an upper surface of a foamed sticking layer formed by laminating a foamed substrate and the adhesive layer for the substrate. After the pair of electrodes were stuck to the sticking surface side of the biological adhesive layer, the sensor portion was installed on the upper surface of the biological adhesive layer. The biosensor was prepared by installing the foamed sheet and the casing on the upper surface of the biological adhesive layer so that the sensor portion is arranged in a space formed of the foamed sheet and the casing.

### (Evaluation of Fluctuation of Power Supply Voltage)

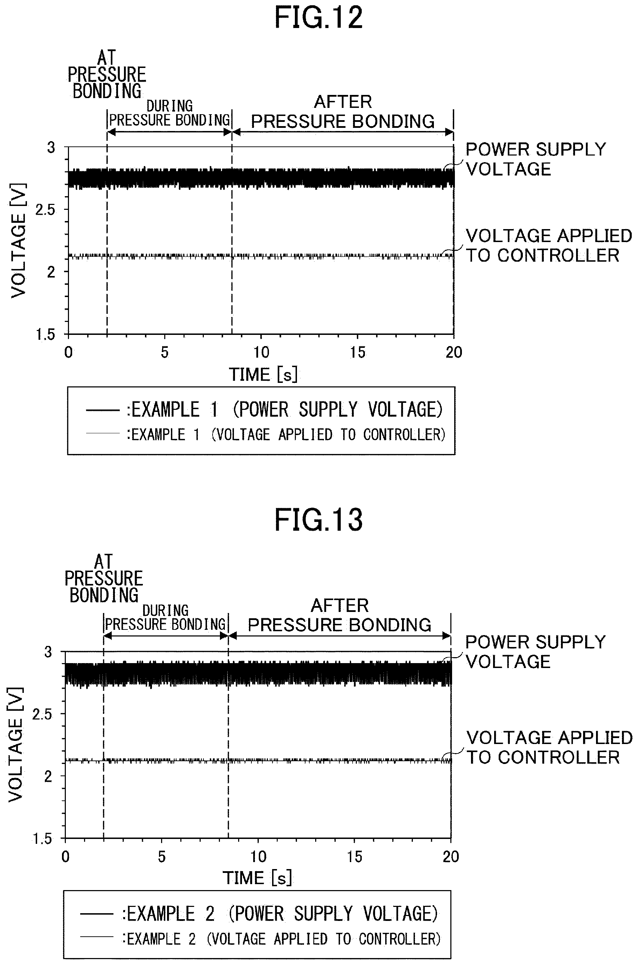

Two seconds after a current started to flow from a coin-shaped battery of the biosensor, a load (pressure: 20 N/cm²) was applied to the casing located above the coin-shaped battery in the affixing direction for about 6.5 seconds, and then the pressure application was stopped. The fluctuation of the power supply voltage during this period was measured. FIG. 12 shows the measurement results of the power supply voltage. In FIG. 12, the power supply voltage of the battery is indicated by a thick solid line, and the voltage applied to the component mounting portion (controller) such as the CPU where the sensor portion is installed is indicated by a thin solid line.

### <Examples 2 - 6>

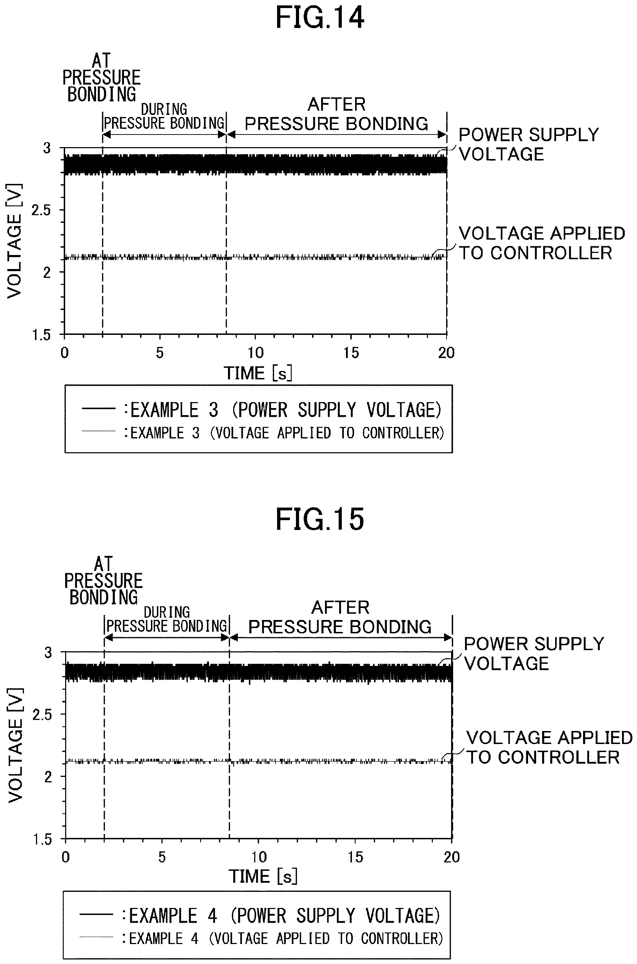

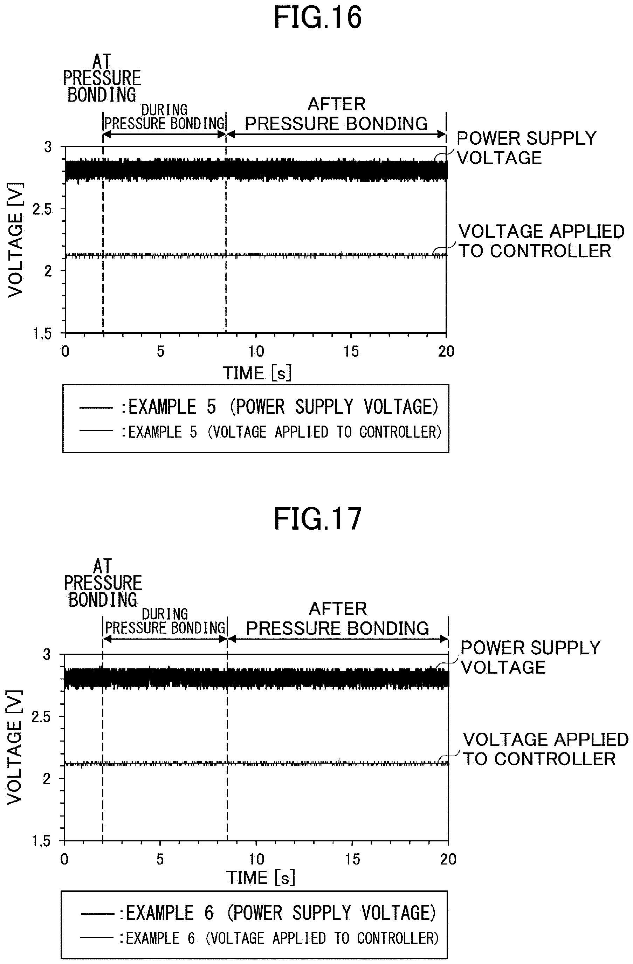

The same procedure as in Example 1 was performed except that the conductive adhesive tape 1 in Example 1 was changed to the following conductive adhesive tapes 2 - 6. FIGS. 13 to 17 show the results of measuring the fluctuation of the power supply voltage of the biosensors prepared with the conductive adhesive tapes 2 - 6 in Examples 2 - 6.
- Conductive adhesive tape 2 (Product name CN4490, manufactured by 3M Japan Limited)
- Conductive adhesive tape 3 (Product name 9725, manufactured by 3M Japan Limited)

- Conductive adhesive tape 4 (Product name 9720S, manufactured by 3M Japan Limited)
- Conductive adhesive tape 5 (Product name No. 7025, manufactured by Teraoka Seisakusho Co., Ltd.)
- Conductive adhesive tape 6 (Product name T4420W, manufactured by Dexerials Corporation)

### <Comparative Examples 1 - 3>

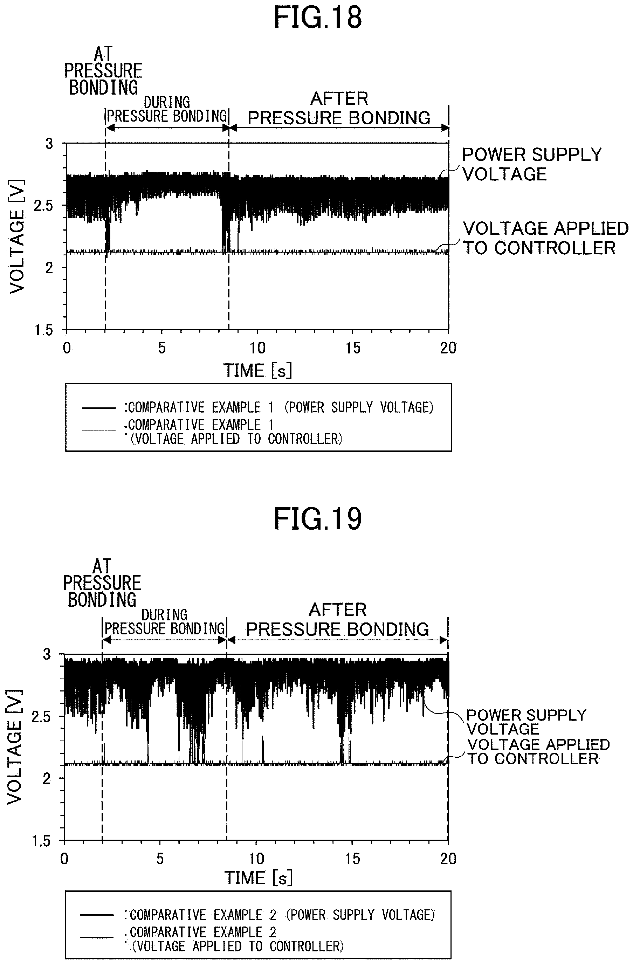

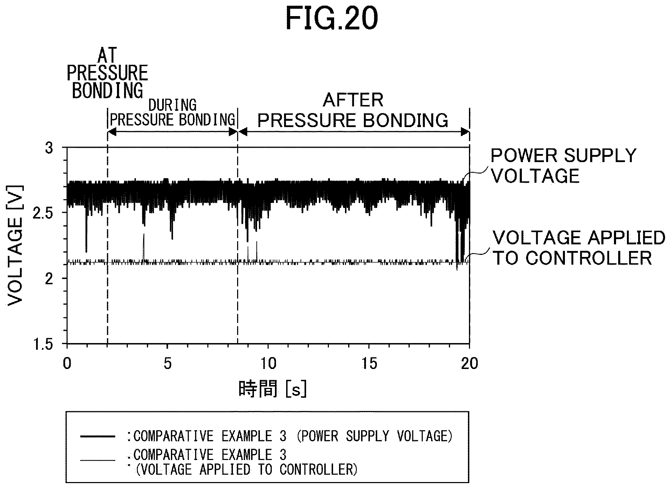

The same procedure as in Example 1 was performed except that the conductive adhesive tape 1 in Example 1 was changed to the following conductive adhesive tapes 7 - 9. FIGS. 18 to 20 show the results of measuring the fluctuation of the power supply voltage of the biosensors prepared with the conductive adhesive tapes 7 - 9 in Comparative Examples 1 - 3.
- Conductive adhesive tape 7 (Product name No. 792, manufactured by Teraoka Seisakusho Co., Ltd.)
- Conductive adhesive tape 8 (Product name X7001, manufactured by 3M Japan Limited)
- Conductive adhesive tape 9 (Product name Al-25DC, manufactured by 3M Japan Limited)

TABLE 1 shows types of the conductive adhesive tapes obtained in respective Examples and Comparative Examples, measurement results of resistance fluctuation values, and evaluation results of voltage stability.

**[TABLE 1]**

| | Type | Substrate | Conductive fine particles | | Conductive fiber | Filler filling rate [wt%] | Thickness of conductive adhesive tape [µm] | Inclination by bending load per unit bending deflection [N/cm] | Resistance fluctuation value [Ω] | Voltage stability |
|---|---|---|---|---|---|---|---|---|---|---|
| | | | Element | Average particle diameter [µm] | Average fiber diameter [µm] | | | | | |
| Ex. 1 | Conductive adhesive tape 1 | Substrate less | Ag | 5 | - | 56 | 50 | 0.02 | 0.06 | A |
| Ex. 2 | Conductive adhesive tape 2 | Nonwoven fabric | Ni | 6 | 0.4 | 26 | 50 | 0.02 | 0.04 | A |
| Ex. 3 | Conductive adhesive tape 3 | Nonwoven fabric | Ni | 4 | 0.7 | 36 | 50 | 0.02 | 0.01 | A |
| Ex. 4 | Conductive adhesive tape 4 | Nonwoven fabric | Ni | 4 | 0.5 | 28 | 30 | 0.01 | 0.01 | A |
| Ex. 5 | Conductive adhesive tape 5 | Substrate less | Ni | 4 | - | 49 | 35 | 0.02 | 0.05 | A |
| Ex. 6 | Conductive adhesive tape 6 | Substrate less | Ni | 6 | - | 57 | 35 | 0.01 | 0.23 | A |
| Comp. ex. 1 | Conductive adhesive tape 7 | Rolled copper foil | Ni | 5 | - | 87 | 90 | 0.08 | 3.20 | B |
| Comp. ex. 2 | Conductive adhesive tape 8 | Woven fabric | Cu | 3 | 1.1 | 40 | 110 | 0.04 | 3.12 | B |
| Comp. ex. 3 | Conductive adhesive tape 9 | Aluminum foil | Ni | 3 | - | 59 | 85 | 0.04 | 1.68 | B |

In Examples 1 - 6, as shown in TABLE 1, the resistance fluctuation value of the conductive adhesive tape was 0.23 Ω or less, and as shown in FIGS. 12 to 17, the power supply voltage was within the range of 2.7 V - 2.9 V, the voltage applied to the controller was about 2.1 V, and the voltage stability was excellent. On the other hand, in Comparative Examples 1 - 3, as shown in TABLE 1, the resistance fluctuation value of the conductive adhesive tape was about 1.7 Ω or more, and as shown in FIGS. 18 to 20, the power supply voltage fluctuated within the range of 2.1 V - 2.8 V, the voltage applied to the controller fluctuated within the range of 2.1 V - 2.5 V, and the voltage stability was poor.

Therefore, in the conductive adhesive tapes of Examples 1 - 6, different from the conductive adhesive tapes of Comparative Examples 1 - 3, fluctuation in resistance can be suppressed by setting the bending load per unit bending deflection to 0.02 N/cm or less. Therefore, in the biosensor according to the present embodiment, when the predetermined conductive adhesive tape is used to connect the battery (cell) and the substrate, an increase in the resistance value can be suppressed, so that the contact impedance of the biosensor with the living body can be maintained low, and the electric signal obtained from the living body can be detected with high sensitivity and stability. Therefore, the biosensor can be effectively used to stably measure an electrocardiogram for a long period of time (for example, 24 hours) continuously while being in close contact with a skin of a subject.

As described above, the embodiments have been described, but the above-described embodiments are presented as examples, and the present invention is not limited to the above-described embodiments. The above-described embodiments can be implemented in various other embodiments, and various combinations, omissions, substitutions, changes, and the like can be made without departing from the scope of the invention. These embodiments and variations thereof are included in the scope and the outline of the invention, and are also included in the scope of the invention and the equivalent scope thereof recited in claims.

The present application claims priority under Japanese Patent Application No. 2020-059657, filed March 30, 2020 with the Japan Patent Office, and the entire contents of Japanese Patent Application No. 2020-059657 are incorporated herein by reference.

### [Reference sings list]

- 1: Biosensor
- 2: Skin
- 10: Biological adhesive layer
- 11: Substrate
- 12: Sticking adhesive layer
- 13: Sensor adhesive layer
- 20: Foamed sheet
- 21: Foamed sticking layer
- 211: Foamed substrate
- 212: Substrate adhesive layer
- 22: Casing adhesive layer
- 30: Casing
- 40: Electrode
- 50: Sensor portion
- 51: Flexible substrate (Resin substrate)
- 52: Sensor Body
- 521: Component mounting portion (controller)
- 522: Battery mounting portion
- 54: Battery
- 55: Positive electrode pattern (first electrode portion)
- 56: Negative electrode pattern (second electrode portion)
- 57: Conductive adhesive tape
- 57A: First conductive adhesive tape
- 57B: Second conductive adhesive tape
- 71A,: 71B Adhesive layer
- 711: Adhesive resin
- 712: Conductive fine particles (Conductive filler)
- 713: Conductive fibers

## Claims

1. A biosensor, which operates by power supplied from a battery, comprising:
an electrode portion positioned on at least a side of one terminal of the battery; and
a conductive adhesive tape having conductivity, which is provided between the one terminal and the electrode portion, wherein
a fluctuation width of a resistance value of the conductive adhesive tape is 1.60 Ω or less in absolute value when an iron ball having a weight of 33 g is dropped vertically from a height of 30 cm to apply a load to a surface of the conductive adhesive tape.

2. The biosensor according to claim 1, wherein
the electrode portion includes a first electrode portion located on a side of a first terminal of the battery, and a second electrode portion located on a side of a second terminal of the battery, and
the conductive adhesive tape includes a first conductive adhesive tape provided between the first terminal and the first electrode portion, and a second conductive adhesive tape provided between the second terminal and the second electrode portion.

3. The biosensor according to claim 1 or 2, wherein
the conductive adhesive tape includes an adhesive layer containing an adhesive resin and conductive fine particles dispersed in the adhesive resin.

4. The biosensor according to claim 3, wherein
the adhesive resin does not include a substrate.

5. The biosensor according to claim 3 or 4, wherein
the conductive adhesive tape includes a conductive fiber, formed by coating a surface of a fiber base material with a conductive material, in the adhesive resin.

6. The biosensor according to claim 5, wherein
an average fiber diameter of the conductive fiber is less than 1.1 um.

7. The biosensor according to any one of claims 1 to 6, wherein
a thickness of the conductive adhesive tape is from 20 um to 80 um inclusive.
